Europäisches Patentamt
European Patent Office
Office européen des brevets

Veröffentlichungsnummer: 0 283 931 A1

# EUROPÄISCHE PATENTANMELDUNG

Anmeldenummer: 88104243.6

Anmeldetag: 17.03.88

Int. Cl.4: C07H 15/252, A61K 31/70

Claims for the following Contracting States: ES + GR.

Priorität: 21.03.87 DE 3709337

Veröffentlichungstag der Anmeldung: 28.09.88 Patentblatt 88/39

Benannte Vertragsstaaten: AT BE CH DE ES FR GB GR IT LI LU NL SE

Anmelder: HOECHST AKTIENGESELLSCHAFT
Postfach 80 03 20
D-6230 Frankfurt am Main 80(DE)

Erfinder: Berscheid, Hans Gerd, Dr.
Rheinlandstrasse 21
D-6231 Schwalbach am Taunus(DE)
Erfinder: Fehlhaber, Hans-Wolfram, Dr.
Thomas-Mann-Strasse 5a
D-6270 Idstein/Taunus(DE)
Erfinder: Kraemer, Hans Peter, Dr.
Birkenweg 16
D-3550 Marburg(DE)
Erfinder: Zilg, Harald, Dr.
Am Kornacker 31
D-3550 Marburg(DE)

## Neue Anthracyclin-Glycoside, ein Verfahren zu ihrer Herstellung und ihre Verwendung als Cytostatika.

Die vorliegende Erfindung betrifft Anthracyclin-Derivate der Formel I

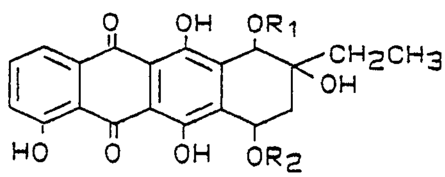

(I)

worin mindestens einer der beiden Substituentenn $R^1$ und $R^2$ ein N-oxidierter Zucker oder eine N-oxidierte Zuckerkombination darstellt, sowie ein chemisches Verfahren zur Herstellung dieser Verbindungen und ihre Verwendung als Arzeimittel zur Behandlung von malignen Tumoren.

EP 0 283 931 A1

**Neue Anthracyclin-Glycoside, ein Verfahren zu ihrer Herstellung und ihre Verwendung als Cytostatika**

Die vorliegende Erfindung betrifft Anthracyclin-Derivate der Formel I

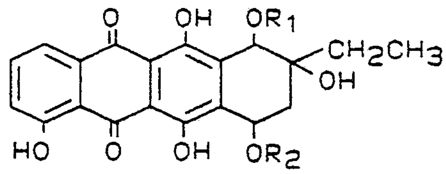

(I)

worin $R^1$ und $R^2$ verschieden sind und einer der beiden Reste den Zuckerbaustein L-Rhodosamin (Roa) der Formel

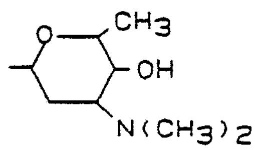

oder dessen N-Oxid der Formel

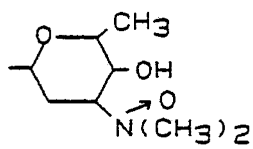

darstellt, während der andere die Zuckerkombination L-Rhodosamin-L-Rhodinose-L-Rhodinose (Roa-Rod-Rod) der Formel

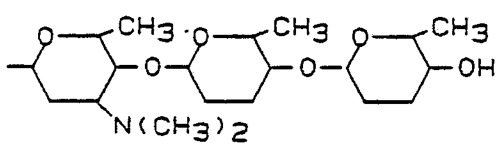

oder L-Rhodosamin-L-Rhodinose-L-Aculose (Roa-Rod-Acu) der Formel

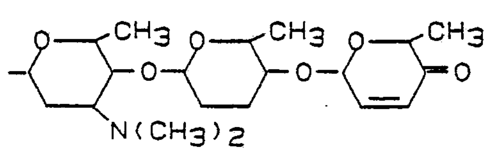

oder L-Rhodosamin-2-desoxy-L-Fucose-L-Cinerulose A (Roa-dF-Cin-A) der Formel

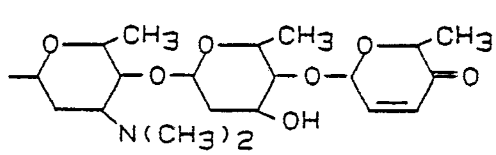

oder L-Rhodosamin-2-desoxy-L-Fucose-L-Cinerulose B (Roa-dF = Cin B) der Formel

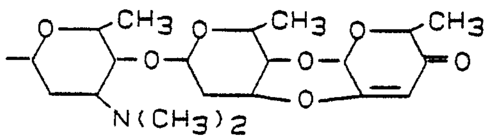

oder L-Rhodosamin-2-Rhodinose-L-Cinerulose A (Roa-Rod-Cin A) der Formel

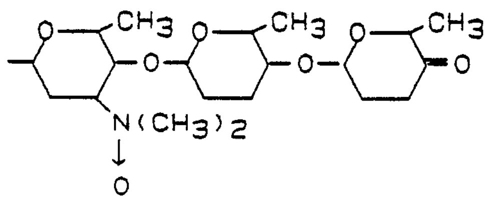

oder L-Rhodosamin-N-oxid-L-Rhodinose-L-Rhodinose (Roa-N-oxid-Rod-Rod) der Formel

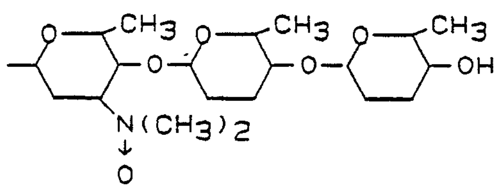

oder L-Rhodosamin-N-oxid-L-Rhodinose-L-Aculose (Roa-N-oxid-Rod-Acu) der Formel

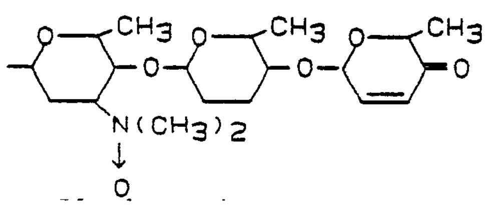

oder L-Rhodosamin-N-oxid-L-Rhodinose-L-Cinerulose A (Roa-N-oxid-Rod-Cin A) der Formel

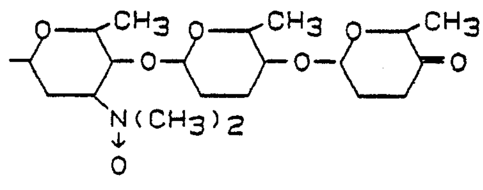

oder L-Rhodosamin-N-oxid-2-desoxy-L-Fucose-L-Cinerulose A (Roa-N-oxid-dF-Cin A) der Formel

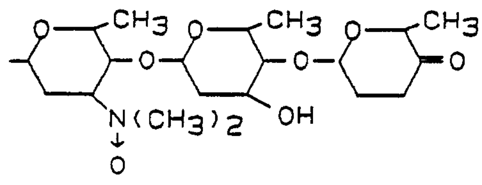

oder L-Rhodosamin-N-oxid-2-desoxy-L-Fucose-L-Cinerulose B (Roa-N-oxid-dF-Cin B) der Formel

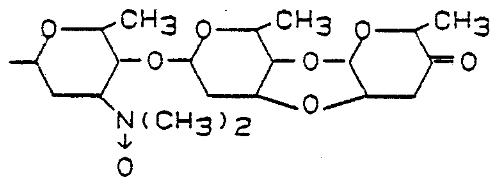

oder L-Rhodosamin-N-oxid-L-Rhodinose-L-Cinerulose A (Roa-N-oxid-Rod-Cin A) der Formel

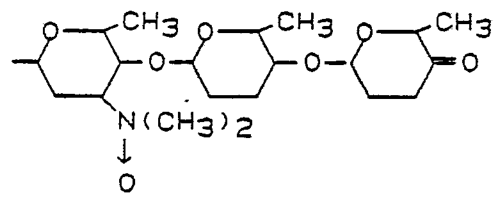

darstellt, mit der Maßgabe, daß mindestens einer der beiden Reste $R^1$ und $R^2$ das entsprechende Zucker-N-oxid darstellt.

Die vorliegende Erfindung betrifft ferner ein Verfahren zur Herstellung der Verbindungen der Formel I, das dadurch gekennzeichnet ist, daß man einer der in EP-A-0 131 942 und EP-A2-0 207 463 beschriebenen Verbindungen der Formel II

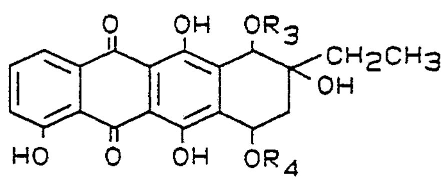

(II)

worin einer der beiden Substituenten $R^3$ und $R^4$ eine der Zuckerkombinationen Roa-Rod-Rod, Roa-Rod-Acu, Roa-dF-CinA, Roa-Rod-CinA, Roa-dF-Rod oder Roa-dF=CinB, der andere den Zuckerbaustein Roa darstellt, wobei CinB Cinerulose B bedeutet und dF=CinB bedeutet, daß die beiden Zuckerbausteine durch eine zusätzliche Ätherbrücke neben der üblichen glykosidischen Bindung verknüpft sind, durch Behandlung mit einem Sauerstoff übertragenden Reagenz in das Aminoxid überführt.

Die N-Oxidierungs-Reaktion wird bei Temperaturen zwischen 0 und 35 °C, vorzugsweise 10-30 °C durchgeführt. Die Reaktionszeit kann mehrere Stunden bis Tage betragen, ist jedoch für jede der Verbindungen unterschiedlich.

Nach erfolgter Reaktion werden die gewünschten Verbindungen aus dem Reaktionsmedium durch Verteilung zwischen wässrigen Pufferlösungen und organischen Lösungsmitteln angereichert und durch Säulenchromatographie oder präparative Schichtchromatographie, vorzugsweise an Kieselgel oder Umkehrphasen, isoliert und gereinigt.

Für diese Umsetzung kommen die folgenden Reagenzien infrage: $H^2O^2$, Persäuren wie z.B. m-Chlorperbenzoesäure, Peressigsäure, Monopermaleinsäure, Trifluorperessigsäure; Hydroperoxide, wie z.B. 2-Hydroperoxyhexafluoro-2-propanol (Ganem et al., Tetrahedron Letters <u>21</u>, 689 (1980)) oder 3-Bromo-4,5-dihydro-5-hydroperoxy-4,4-dimethyl-3,5-diphenyl-2H-pyrazol (Baumstark et al., Tetrahedron Letters <u>22</u>, 4591 (1981)).

Die Umsetzung kann ebenfalls mikrobiell mit geeigneten Mikroorganismen oder daraus isolierten Enzymen, z.B. mit Streptomyces purpurascens (DSM 2658), durchgeführt werden.

Bevorzugt wird die Umsetzung mit $H^2O^2$ in einem geeigneten Lösungsmittel, z.B. in einem niederen Alkanol oder Keton, in Wasser oder wäßrigen Pufferlösungen bei einer Temperatur zwischen 10 und 70 °C und einem pH-Wert zwischen 3 und 8 durchgeführt.

Man erhält durch das erfindungsgemäße Verfahren z.B. die folgenden Verbindungen, bei denen es sich um rote, i.a. amorphe Substanzen handelt, die leicht löslich in Methanol, Ethylacetat, Chloroform und Toluol, unlöslich hingegen in Wasser und Hexan sind und die dünnschichtchromatographisch an Kieselgel 60 $F^{254}$-(Merck) vorzugsweise mit dem Laufmittelsystem Chloroform/Methanol/Eisessig/Wasser/Triethylamin = 80/10/10/2/0,01 charakterisiert werden können.

Nachweis der Bildung, quantitative Verfolgung der Reaktion und Reinheitsprüfung kann vorteilhaft auch

mit analytischer HPLC erfolgen:
Säule: Lichrospher ®100 RP-18.5 µm. 4 × 250 mm (Merck)
Mobile Phase: 14 % (Vol) $CH_3CN$-86 % (Vol) Pufferlösung
Pufferlösung: 20 % (Vol) Triethylamin in Wasser mit ortho-Phosphorsäure (85 %ig) auf pH 3 eingestellt
Fluß: 0.5 ml/min
Detektion: 254 nm oder 495 nm
Retentionszeit:
Cytorhodin S ca. 55 min
Cytorhodin S-N-oxid ca. 80 min
Cytorhodin S-bis-N-oxid ca. 140 min.

Die erfindungsgemäßen Verbindungen besitzen zytostatische Wirksamkeit, d.h. therapeutische Wirkung gegen Tumoren, insbesondere maligne Tumoren bei Tieren und Menschen.

Die Verbindungen und ihre Säureadditionssalze können daher als Medikamente zur Behandlung von Tumoren verwendet werden. Die Verbindungen können auf verschiedene Weise in Abhängigkeit von der Dosierungsform verabreicht werden.

Normalerweise werden die Verbindungen oder ihre Säureadditionssalze, z.B. mit D-Gluconsäure, mit pharmazeutisch üblichen Trägerstoffen oder Verdünnungsmitteln vermischt verabreicht. So können sie z.B. einzeln oder in Mischung zusammen mit Trägerstoffen wie Maltose oder Lactose oder als nicht-toxische Komplexe, z.B. als Desoxyribonukleinsäure-Komplex, verabreicht werden.

Eine typische Verabreichungsart ist die Injektion einer Lösung der erfindungsgemäßen Verbindung in destilliertem Wasser oder in physiologischer Kochsalzlösung. Die Lösungen können intraperitoneal, intravenös oder intraarteriell injiziert werden.

Tagesdosis und Einheitsdosis können aus Tierversuchen und auch aus in vitro-Tests in der Weise festgesetzt werden, daß die Gesamtdosis, die kontinuierlich oder in Abständen verabreicht wird, einen vorher festgelegten Bereich nicht überschreitet. So beträgt die Gesamtdosis für einen Behandlungszyklus etwa 0,5-5 mg/kg Körpergewicht. Diese Dosis kann in entsprechenden Bruchteilen über einen Zeitraum von 7 Tagen verabreicht werden. Es ist jedoch klar, daß konkrete Dosen für die Behandlung von Mensch oder Tier individuell festgelegt werden können in Abhängigkeit von der jeweiligen Situation des Patienten, z.B. Alter, Körpergewicht, Geschlecht, Empfindlichkeit, Nahrung, Zeitpunkt der Verabreichung, weiteren verabreichten Medikamenten, körperlichem Zustand der Patienten und Schwere der Erkrankung.

Die Herstellung der erfindungsgemäßen Verbindungen wird im folgenden Beispiel beschrieben:

**Beispiela**)

Ca. 400 mg einer Verbindung der oben genannten Formel I, worin $R^1$ L-Rhodosamin (Roa) und $R^2$ die Zuckerkombination L-Rhodosamin-2-desoxy-L-Fucose-L-Cinerulose B (Roa-dF = Cin B) darstellt, mit der Bezeichnung

"Cytorhodin S", wurden in 100 ml Methanol gelöst, bei Raumtemperatur unter Lichtausschluß gerührt und mit 4 ml Wasserstoffperoxid (30 %) versetzt. Die Zugabe von $H_2O_2$ wurde nach 27 Stunden wiederholt. Die Umsetzung wurde mit analytischer HPLC verfolgt.

Nach insgesamt 51 Stunden wurde überschüssiges Reagenz durch Zugabe von Palladiummohr zersetzt und die Reaktion damit beendet.

Die Reaktionslösung wurde über eine Glasfritte, die mit einer Schicht von Celite® bedeckt war, abgesaugt und am Rotationsverdampfer unter Vakuum eingeengt. Der Rückstand wurde mit 30 ml wässrigen Na-Acetatpuffer pH 3,5 aufgenommen, 0,5 ml 0,1 M EDTA-Lösung zugesetzt und 8 mal mit ca. 20 ml $CHCl_3$ extrahiert. Die vereinigten $CHCl_3$-Extrakte wurden mit wenig Wasser gewaschen und i.V. eingedampft. Der Rückstand betrug 244 mg.

Nach Umstellen des pH-Wertes auf 7,0 mit 1 N NaOH wurden durch zweimalige Extraktion mit $CHCl_3$ weitere 44 mg Produkt gewonnen. Nach analytischer HPLC enthalten beide Extrakte hauptsächlich zwei neugebildete Verbindungen neben wenig nicht umgesetztem Edukt Cytorhodin S.

Reaktionsprodukte:

| | % N-Oxid | Bis-N-Oxid | Cytorhodin S |
|---|---|---|---|
| Hauptextrakt | 65 | 30 | 2 |
| Nachextrakt | 25 | - | 10 |

Der Nachextrakt enthält noch zu ca. 35 % eine noch nicht identifizierte Verbindung mit einer Retentionszeit von ca. 110 min. b) Isolierung der Reaktionsprodukte durch "reversed phase" HPLC:

ca. 240 mg des Hauptextrakts wurden in 20 ml der mobilen Phase $CHCl_3$/MeOH/10 % Ammoniumacetat in $H_2O$ 150:1050: 375 gelöst und in einer Stahlsäule 3,2 × 25 cm an 110 g Lichrospher® 100 RP-18, 10 μ - (Merck) bei einem Fluß von 2 ml/min. chromatographiert. Mit einem Durchflußphotometer wurde bei der Wellenlänge 490 nm der Verlauf der Elution verfolgt. Die Fraktionen von 5 ml wurden nach Beurteilung durch DC zusammengefaßt und aufgearbeitet. Dazu wurde mit der Hälfte des Volumens an Wasser verdünnt, $CHCl_3$ bis zur Phasentrennung zugesetzt und die abgetrennte $CHCl_3$-Phase mit $H_2O$ gewaschen, über $Na_2SO_4$ getrocknet und im Vakuum eingedampft. Erhalten wurden:

| Fraktion | mg | Verbindung |
|---|---|---|
| 38-47 | 40 | Cytorhodin S-bis-N-oxid |
| 100-150 | 90 | Cytorhodin S-N-oxid |

Cytorhodin S-N-oxid (Formel I mit $R^1$ = Roa-N-oxid und $R^2$ = Roa-dF=CinB) : 1H-NMR Fig. 1
Absorptions-Spektrum
235 (4,6); 254 (4,3); 293 (3,86); 496 (4,17)
$C_{48}H_{64}N_2O_{18}$, M ber 956 (FAB-MS bestätigt)
Cytorhodin S-bis-N-oxid (Formel I mit $R^1$ = Roa-N-Oxid und $R^2$ = Roa-N-oxid-dF=CinB) : 1H-NMR Fig. 2
Absorptions-Spektrum
235 (4,6); 252 (4,3); 294 (3,87); 495 (4,18)
$C_{48}H_{64}N_2O_{19}$, M ber 972 (FAB-MS bestätigt)

Die Identifizierung der Verbindungen im vorstehenden Beispiel erfolgte unter den anschließend beschriebenen Meßbedingungen:

Die Protonen-Resonanzspektren ($^1$H-NMR-Spektren) wurden auf einen HX-270 BRUKER Fourier-Transform Kernresonanzspektrometer bei 270 MHZ gemessen. Die Konzentrationen betrugen 2-4 mg/0,5 ml 99,8 % $CDCl_3$; die Lösungen wurden sofort nach Herstellung mit 0,1 ml 5 %iger $Na_2CO_3$ 99,5 % $D_2O$ geschüttelt.

Die in den Abbildungen mit einem Stern versehenen Signale rühren her von niedermolekularen Verunreinigungen im o/oo-Bereich und von Lösungsmittelresten.

Die Massenspektren wurden auf dem Massenspektrometer MS-902 S, AEI, unter Verwendung einer FAB-(Fast-Atom-Bombardment-)Ionenquelle gemessen. Die Substanzen wurden in einer Matrix von 3-Nitrobenzylalkohol oder Thioglycerin in die Ionenquelle eingebracht, teilweise unter Zusatz von Ammoniumchlorid.

Die Absorptionsspektren wurden im Bereich 200-700 nm in 10 % 1 N HCl in Methanol gemessen.

Die Substanzkonzentration betrug 10-30 mg/l; angegeben werden die Absorptionsmaxima in nm und die molaren Extinktionskoeffizienten (log ε).

## Ansprüche

1. Verbindungen der Formel I

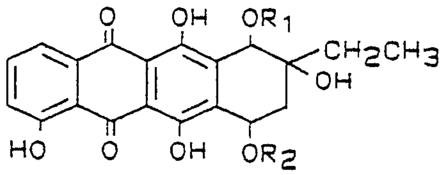

( I )

worin $R^1$ und $R^2$ verschieden sind und einer der beiden Reste den Zuckerbaustein L-Rhodosamin (Roa) der Formel

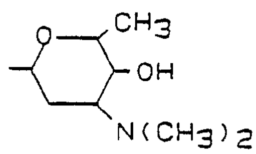

oder dessen N-Oxid der Formel

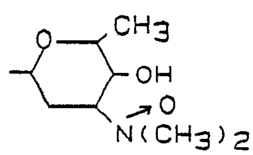

darstellt, während der andere die Zuckerkombination L-Rhodosamin-L-Rodinose-L-Rhodinose (Roa-Rod-Rod) der Formel

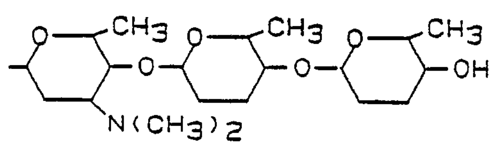

oder L-Rhodosamin-L-Rhodinose-L-Aculose (Roa-Rod-Acu) der Formel

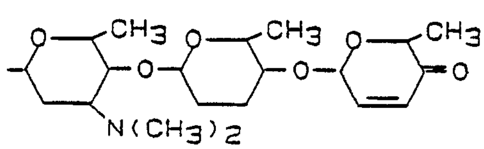

oder L-Rhodosamin-2-desoxy-L-Fucose-L-Cinerulose A (Roa-dF-Cin-A) der Formel

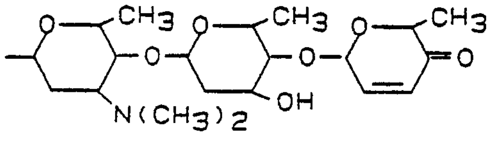

oder L-Rhodosamin-2-desoxy-L-Fucose-L-Cinerulose B (Roa-dF = Cin B) der Formel

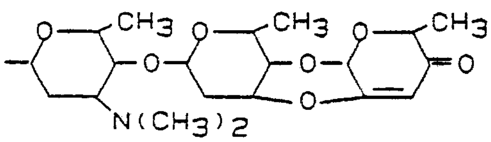

oder L-Rhodosamin--L-Rhodinose-L-Cinerulose A (Roa-Rod-Cin A) der Formel

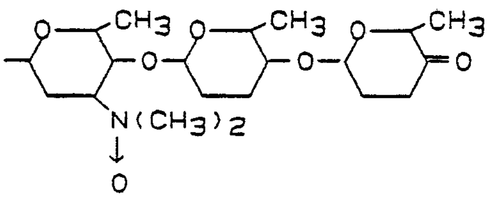

oder L-Rhodosamin-N-oxid-L-Rhodinose-L-Rhodinose (Roa-N-oxid-Rod-Rod) der Formel

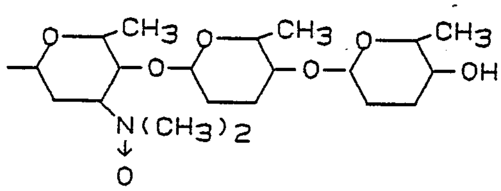

oder L-Rhodosamin-N-oxid-L-Rhodinose-L-Aculose (Roa-N-oxid-Rod-Acu) der Formel

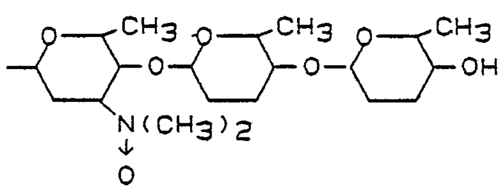

oder L-Rhodosamin-N-oxid-L-Rhodinose-L-Cinerulose A (Roa-N-oxid-Rod-Cin A) der Formel

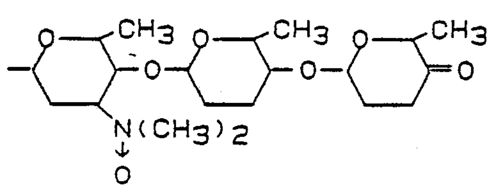

oder L-Rhodosamin-N-oxid-2-desoxy-L-Fucose-L-Cinerulose A (Roa-N-oxid-dF-Cin A) der Formel

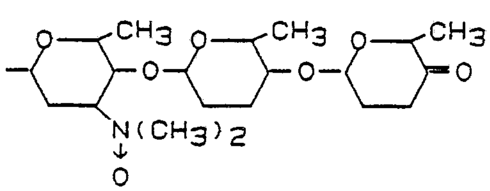

oder L-Rhodosamin-N-oxid-2-desoxy-L-Fucose-L-Cinerulose B (Roa-N-oxid-dF-Cin B) der Formel

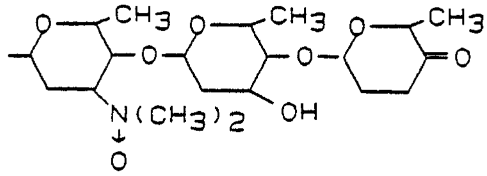

oder L-Rhodosamin-N-oxid-L-Rhodinose-L-Cinerulose A (Roa-N-oxid-Rod-Cin A) der Formel

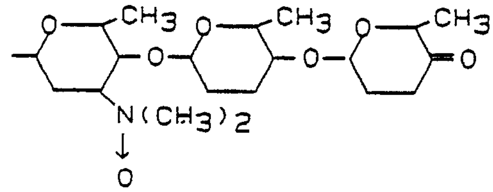

darstellt, mit der Maßgabe, daß mindestens einer der beiden Reste $R^1$ und $R^2$ das entsprechende Zucker-N-oxid darstellt.

2. Verbindungen gemäß Anspruch 1, dadurch gekennzeichnet, daß $R^1$ das L-Rhodosamin-N-oxid darstellt.

3. Verbindung gemäß Anspruch 2, dadurch gekennzeichnet, daß $R^2$ die Zuckerkombination Roa-dF = CinB darstellt.

4. Verbindungen gemäß Anspruch 2, dadurch gekennzeichnet, daß $R^2$ die N-oxidierte Zuckerkombination Roa-N-oxid-dF = CinB darstellt.

5. Verfahren zur Herstellung einer Verbindung gemäß Anspruch 1, dadurch gekennzeichnet, daß man eine Verbindung der Formel II

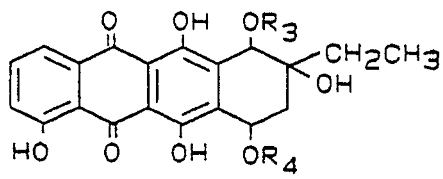

(I I)

worin einer der beiden Substituenten $R^3$ und $R^4$ den Zuckerbaustein Roa und der andere eine der Zuckerkombinationen Roa-Rod-Rod, Roa-Rod-Acu, Roa-dF-CinA, Roa-Rod-CinA, Roa-dF-Rod oder Roa-dF = CinB bedeutet, mit einem Sauerstoff übertragenden Reagenz umsetzt.

6. Verfahren nach Anspruch 5, dadurch gekennzeichnet, daß man die Umsetzung in einem geeigneten Lösungsmittel bei einer Temperatur zwischen 0 und 35 °C durchführt.

7. Verfahren nach Anspruch 6, dadurch gekennzeichnet, daß man die Umsetzung mit Wasserstoffperoxid in einem niederen Alkanol oder Keton, in Wasser oder wäßrigen Pufferlösungen bei einer Temperatur zwischen 10 und 30 °C und einem pH-Wert zwischen 3 und 8 durchführt.

8. Arzneimittel, dadurch gekennzeichnet, daß es eine Verbindung gemäß Anspruch 1 neben üblichen Hilfs-und/oder Trägerstoffen enthält.

9. Verwendung einer Verbindung gemäß Anspruch 1, zur Herstellung eines Arzneimittels mit cytostatischer Wirkung.

Patentansprüche für folgende Vertragsstaaten: ES, GR

1. Verbindungen der Formel I

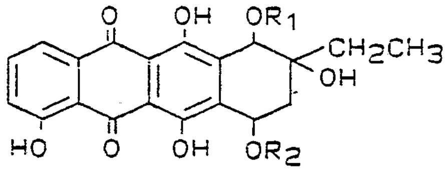

( I )

worin $R^1$ und $R^2$ verschieden sind und einer der beiden Reste den Zuckerbaustein L-Rhodosamin (Roa) der Formel

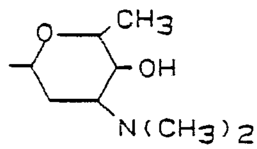

oder dessen N-Oxid der Formel

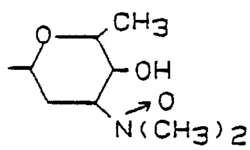

darstellt, während der andere die Zuckerkombination L-Rhodosamin-L-Rodinose-L-Rhodinose (Roa-Rod-Rod) der Formel

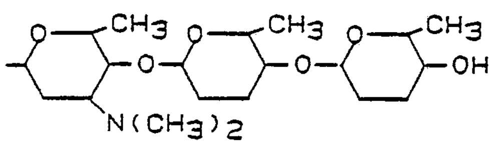

oder L-Rhodosamin-L-Rhodinose-L-Aculose (Roa-Rod-Acu) der Formel

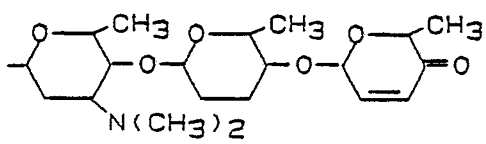

oder L-Rhodosamin-2-desoxy-L-Fucose-L-Cinerulose A (Roa-dF-Cin-A) der Formel

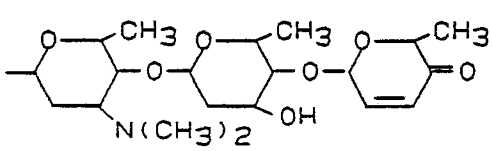

oder L-Rhodosamin-2-desoxy-L-Fucose-L-Cinerulose B (Roa-dF = Cin B) der Formel

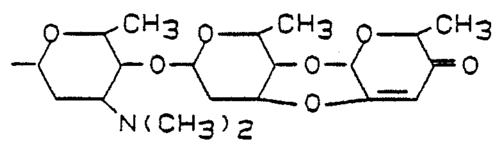

oder L-Rhodosamin-L-Rhodinose-L-Cinerulose A (Roa-Rod-Cin A) der Formel

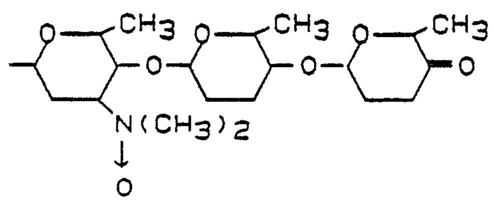

oder L-Rhodosamin-N-oxid-L-Rhodinose-L-Rhodinose (Roa-N-oxid-Rod-Rod) der Formel

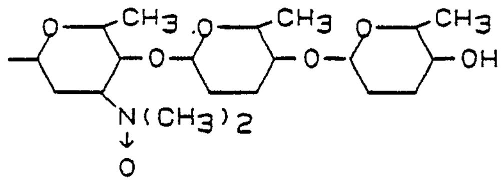

oder L-Rhodosamin-N-oxid-L-Rhodinose-L-Aculose (Roa-N-oxid-Rod-Acu) der Formel

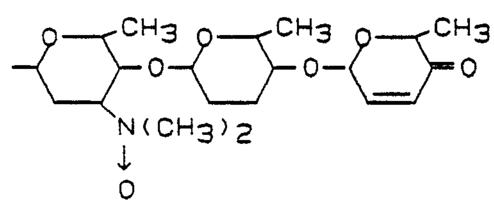

oder L-Rhodosamin-N-oxid-L-Rhodinose-L-Cinerulose A (Roa-N-oxid-Rod-Cin A) der Formel

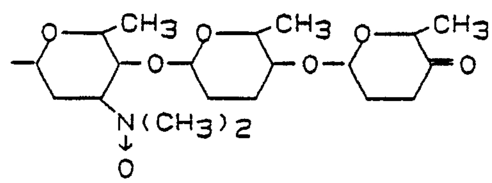

oder L-Rhodosamin-N-oxid-2-desoxy-L-Fucose-L-Cinerulose A (Roa-N-oxid-dF-Cin A) der Formel

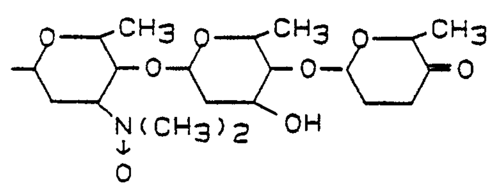

oder L-Rhodosamin-N-oxid-2-desoxy-L-Fucose-L-Cinerulose B (Roa-N-oxid-dF-Cin B) der Formel

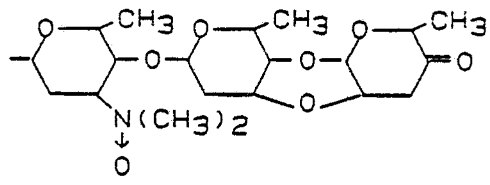

oder L-Rhodosamin-N-oxid-L-Rhodinose-L-Cinerulose A (Roa-N-oxid-Rod-Cin A) der Formel

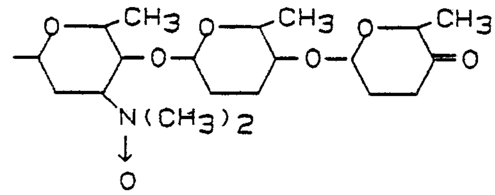

darstellt, mit der Maßgabe, daß mindestens einer der beiden Reste $R^1$ und $R^2$ das entsprechende Zucker-N-oxid darstellt, dadurch gekennzeichnet, daß man eine Verbindung der Formel II

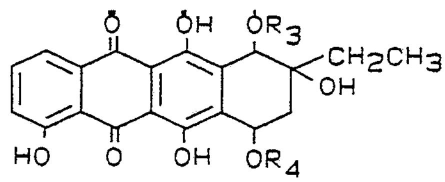

(II)

worin einer der beiden Substituenten $R^3$ und $R^4$ den Zuckerbaustein Roa und der andere eine der Zuckerkombinationen Roa-Rod-Rod, Roa-Rod-Acu, Roa-dF-CinA, Roa-Rod-CinA, Roa-dF-Rod oder Roa-dF=CinB bedeutet, mit einem Sauerstoff übertragenden Reagenz umsetzt.

2. Verfahren nach Anspruch 1, dadurch gekennzeichnet, daß man die Umsetzung in einem geeigneten Lösungsmittel bei einer Temperatur zwischen 0 und 35 °C durchführt.

3. Verfahren nach Anspruch 1, dadurch gekennzeichnet, daß man die Umsetzung mit Wasserstoffperoxid in einem niederen Alkanol oder Keton, in Wasser oder wäßrigen Pufferlösungen bei einer Temperatur zwischen 10 und 30 °C und einem pH-Wert zwischen 3 und 8 durchführt.

4. Verfahren nach den Ansprüchen 1 - 3, dadurch gekennzeichnet, daß $R^1$ in Formel 1 das L-Rhodosamin-N-oxid darstellt.

5. Verfahren nach Anspruch 4, dadurch gekennzeichnet, daß $R^2$ die Zuckerkombination Roa-dF=Cin B darstellt.

6. Verfahren nach Anspruch 4, dadurch gekennzeichnet, daß $R^2$ die N-oxidierte Zuckerkombination Roa-N-oxid-dF=Cin B darstellt.

7. Verwendung einer Verbindung gemäß Anspruch 1 zur Herstellung eines Arzneimittels mit cytostatischer Wirkung.

Europäisches Patentamt

# EUROPÄISCHER RECHERCHENBERICHT

Nummer der Anmeldung

EP 88 10 4243

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 207 463 (HOECHST)<br>* Ansprüche 1,11 *<br>--- | 1,9 | C 07 H 15/252<br>A 61 K 31/70 |
| A,D | EP-A-0 131 942 (HOECHST)<br>* Ansprüche 1,9 *<br>--- | 1,9 | |
| A | JOURNAL OF THE AMERICAN CHEMICAL SOCIETY, Band 91, Nr. 26, 17. Dezember 1969, Seiten 7505-7506; A.K. MALLAMS: "The megalomicins. I. D-rhodosamine, a new dimethylamino sugar"<br>* Seite 7505, Spalte 1, Zeile 52 - Seite 7506, Spalte 1, Zeile 5 *<br>----- | 1 | |
| | | | **RECHERCHIERTE SACHGEBIETE (Int. Cl.4)** |
| | | | C 07 H 15/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 15-06-1988 | BRENNAN J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)